# EUROPÄISCHE PATENTSCHRIFT

(11) **EP 2 008 707 B1**
(45) Veröffentlichungstag und Bekanntmachung des Hinweises auf die Patenterteilung: **06.10.2010**
(21) Anmeldenummer: 08009626.6
(22) Anmeldetag: 27.05.2008
(51) Int. Cl.: B01F 11/00, B01F 13/00, A61F 2/46, B01F 13/06, B01F 15/00

(54) **Misch- und Applikationsvorrichtung von Mischgut, insbesondere von Knochenzement**
Mixing and application device of a mixture, in particular of bone cement
Dispositif de mélange et d'application d'un mélange, particulièrement de ciment osseux

(30) Priorität: 04.06.2007 DE 102007026034
(43) Veröffentlichungstag der Anmeldung: 31.12.2008
(73) Patentinhaber: aap Biomaterials GmbH, 64807 Dieburg (DE)
(72) Erfinder: Sattig, Christoph, 64807 Dieburg (DE); Stirnal, Volker, 64807 Dieburg (DE)
(74) Vertreter: Herden, Andreas F.

(56) Entgegenhaltungen:
- EP-A- 0 882 436
- EP-A- 1 278 488
- WO-A-2004/100771
- US-A- 5 328 262
- US-A- 5 501 520

## Beschreibung

Die Erfindung bezieht sich auf eine Vorrichtung zum Mischen und Applizieren von Mischgut, insbesondere medizinischem Zement, und umfasst einen Kartuschenzylinder mit einem Mischguthohlraum, einem Befüllende und einem Austrittsende, wobei das Befüllende und das Austrittsende sich an unterschiedlichen Enden des Kartuschenzylinders befinden, einem Mischer mit einer Betätigungsstange, einem Mischpaddel und einem Griff, einen Kolben zum Verschließen des Mischguthohlraums am Befüllende während des Mischens und zum Herauspressen des Mischgutes durch das Austrittsende während des Applizierens, und ein Halteelement zum zeitweiligen Befestigen des Kolbens am Befüllende.

Eine derartige Vorrichtung ist aus der EP 1 278 488 B1 bekannt. Die Vorrichtung dient zur Zubereitung einer pastösen, fließfähigen Masse aus einer flüssigen Komponente und einer pulverförmigen Komponente, die in Mischung zeitverzögert aushärten. Im Falle von medizinischem Zement, auch Knochenzement genannt, müssen der Mischvorgang und die Applikation des medizinischen Zements unter sterilen Bedingungen erfolgen, was zu gewissen Zwängen bei der Gestaltung der Vorrichtung führt. Auch wenn die Vorrichtung nur einmal gebraucht wird und dann entsorgt wird, müssen alle Bauteile für die Sterilisation gut zugänglich sein. Aus diesem Grund werden möglichst einfache Bauformen gewünscht. Ein weiteres Erfordernis bei derartigen Geräten ist die einfache Handhabung, um der Gefahr von Fehlern zu entgehen.

Die vorbekannte Vorrichtung (EP 1 278 488 B1) weist eine Verschlusskappe auf, um den Kolben am Befüllende des Kartuschenzylinders zu verriegeln, bevor mit dem Mischvorgang begonnen wird. Wenn das Mischgut appliziert werden soll, muss die Verschlusskappe entfernt werden, um den Kolben zu entriegeln. Die Verschlusskappe stellt somit ein Halteelement zum zeitweiligen Befestigen des Kolbens am Befüllende dar, das entfernt werden muss.

WO 2004/100771 A2 beschreibt eine Mischkartusche zur Zubereitung von Knochenzement, bei der ein proximales Ende sowohl zum Befüllen des Zylinders als auch zum Anbringen eines Applikatordüsenrohres ausgebildet ist. Das entgegengesetzte, distale Ende wird durch einen Kolben verschlossen, der mittels eines Verriegelungsgliedes während der Vorgänge des Einfüllens und des Mischens an der Zylinderwand befestigt ist und zum Applizieren der Mischung aus seiner Verriegelung gelöst wird, um gegen das proximale Ende verschoben zu werden. Da das proximale Ende sowohl zur Befüllung als auch zur Abgabe des Mischgutes benutzt wird, muss die Verschlusskappe am proximalen Ende von der Mischfunktion zur Abgabefunktion der Mischung umgerüstet werden, was gewisse Handhabungen erforderlich macht, bei denen auch Fehler unterlaufen können.

Bei einer weiteren bekannten Mischvorrichtung für Knochenzement (US-A 5,328,262 und 5,501,520) werden am vorderen Ende des Mischzylinders die zu mischenden Komponenten zugegeben, während das hintere Ende durch einen Kolben verschlossen ist, der in der Zylinderwand durch Greifmittel gehalten wird. Das vordere Ende wird durch eine Kappe verschlossen, die eine Öffnung zum Durchgriff eines Mischpaddels und eine weitere Öffnung zum Ansatz eines Absaugstutzens bzw. eines Abgaberohres aufweist. Auch hier bedarf es Handhabungen zur Umrüstung zwischen Füllung und Abgabe von Mischgut.

Der Erfindung liegt die Aufgabe zugrunde, eine Misch- und Applikationsvorrichtung der eingangs angegebenen Art in der Konstruktion und der Handhabung zu vereinfachen.

Zur Lösung dieser Aufgabe ist das Halteelement als Rastring mit radial außenseitigen und innenseitigen Rastmitteln ausgebildet. Um den Rastring zu halten, weist das Befüllende des Kartuschenzylinders erste Gegenrastmittel auf, die mit den außenseitigen Rastmitteln des Rastrings zusammenwirken, wenn der Kolben den Mischguthohlraum verschließt. Der Kolben weist ferner zweite Gegenrastmittel auf, die mit den innenseitigen Rastmitteln des Rastrings zusammenwirken, um den Kolben während des Mischens zu halten, jedoch bei Beginn des Applikationsvorgangs freizugeben, so dass der Kolben mittels eines Applikators vorgeschoben und das Mischgut durch das Austrittsende des Kartuschenzylinders herausgepresst werden kann.

Das Zusammenwirken von Rastring mit dem Befüllende des Kartuschenzylinders einerseits und dem Kolben andererseits ist derart, dass der Kolben mit angebrachtem Rastring von Hand in das Befüllende des Kartuschenzylinders einrastend montiert werden kann, der Rastsitz zwischen Rastring und Kolben jedoch so stark ist, dass bei dieser Montage von Hand der Kolben nicht in das Innere des Mischguthohlraums verschoben wird. Erst der Applikator ist in der Lage, die Haltekraft der Verrastung zwischen Rastring und Kolben zu überwinden, um den Kolben alsdann zum Herauspressen des Mischgutes aus dem Mischguthohlraum zu nutzen.

Der Kolben weist eine ringförmige Aufnahmenut für den Rastring auf, der mit einem Ringabschnitt in diese Aufnahmenut einschnappend montiert wird. Zu diesem Zweck ist die Rastnutwand nachgiebig gestaltet und weist Rasthaken auf, die infolge der Nachgiebigkeit der Wand federnd nachgeben können. Die Nachgiebigkeit der Wand wird durch Schlitze in der Kolbenwand im Bereich der Rastnut erzeugt, wobei die Länge der Schlitze dem Konstrukteur ein Mittel in die Hand gibt, die federnde Nachgiebigkeit der Rasthaken auf das richtige Maß einzustellen. Beispielsweise wird die Rasthaltekraft des Rastringes am Kolben stärker gewählt als die Kraft, die zur Montage des Kolbens samt Rastring am Befüllende des Kartuschenzylinders benötigt wird. Dadurch wird sichergestellt, dass bei der Montage des Kolbens samt Rastring am Befüllende des Kartuschenzylinders nicht unbeabsichtigt der Kolben zu weit ins Innere des Mischguthohlraums gedrückt wird.

Um den Kolben mittels des Rastrings am Befüllende des Kartuschenzylinders zu verrasten, weist das Befüllende des Kartuschenzylinders einen Flanschring auf, dessen radiale Ausdehnung größer ist als die Wandung des Kartuschenzylinders. Dadurch wird ein Ringhohlraum mit gegenüber dem Mischguthohlraum größeren Durchmesser gebildet, und dieser Ringhohlraum wird durch hakenförmige Vorsprünge eingeengt, hinter die sich eine ringförmige Hakenausbildung des Rastringes legt, wenn der Kolben mit angebrachtem Rastring in das Befüllende des Kartuschenzylinders gedrückt wird.

Ein Ausführungsbeispiel der Erfindung wird anhand der Zeichnung beschrieben. Dabei zeigt:
- Fig. 1: die Einzelteile der Misch- und Appliziervorrichtung in auseinandergezogener Darstellung,
- Fig. 2: einen Rastring, durchschnitten,
- Fig. 3: eine vergrößerte Darstellung des Rastringquerschnitts,
- Fig. 4: einen Längsschnitt durch die Misch- und Appliziervorrichtung,
- Fig. 5: eine vergrößerte Darstellung des Befüllendes der Vorrichtung,
- Fig. 6: eine vergrößerte Darstellung des Kolbens der Vorrichtung und
- Fig. 7: eine nochmals vergrößerte Schnittdarstellung des Eingriffs des Rastringes am Befüllende und am Kolben der Vorrichtung.

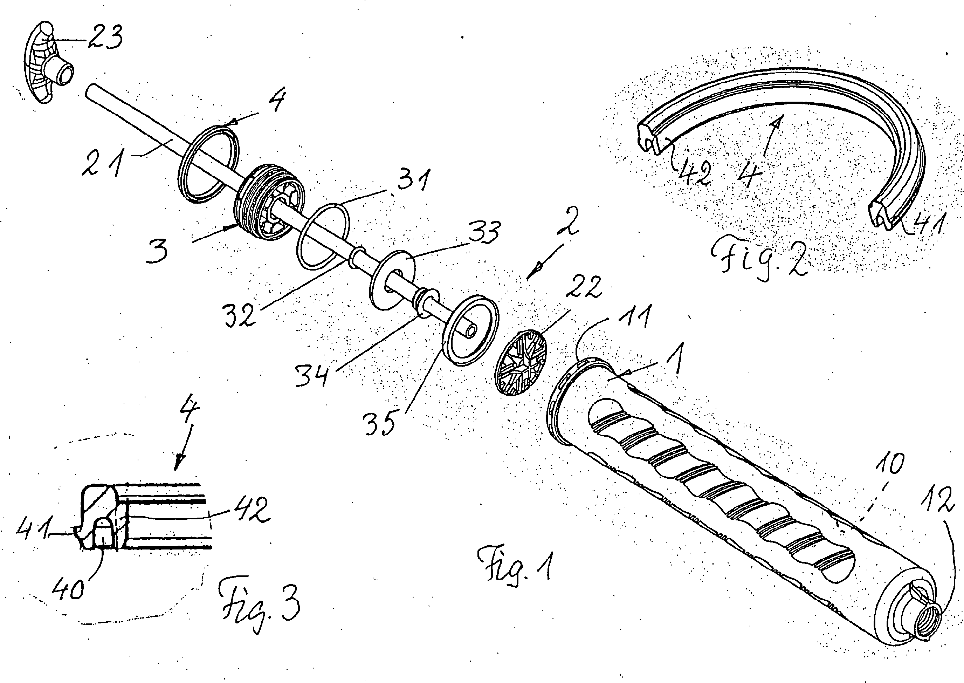

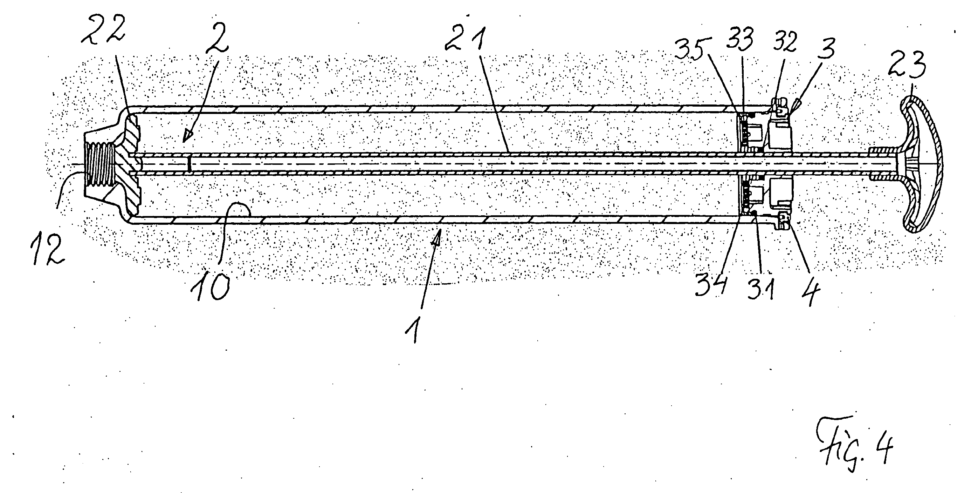

Fig. 1 zeigt die Einzelteile der Vorrichtung und Fig. 4 den Zusammenbau. Als Hauptteile der Vorrichtung sind zu nennen ein Kartuschenzylinder 1, ein Mischer 2, ein Kolben 3 und ein Rastring 4. Der Kartuschenzylinder 1 weist einen Mischguthohlraum 10, ein Befüllende 11 und ein Austrittsende 12 auf. Zum Austrittsende gehört ein nicht dargestellter Schraubverschluss, auch Boden genannt, der nach dem Mischen des Mischgutes gegen ein Applizierrohr, auch Schnorchel genannt, ausgetauscht wird.

Der Mischer 2 umfasst eine Betätigungsstange 21, ein Mischpaddel 22 am vorderen Ende und einen Griff 23 am hinteren Ende. Das Mischpaddel 22 weist sektorförmige Durchlassöffnungen auf, durch welche das Mischgut beim Drehen und Hin- und Herbewegen der Betätigungsstange hindurch gepresst und dadurch gemischt wird.

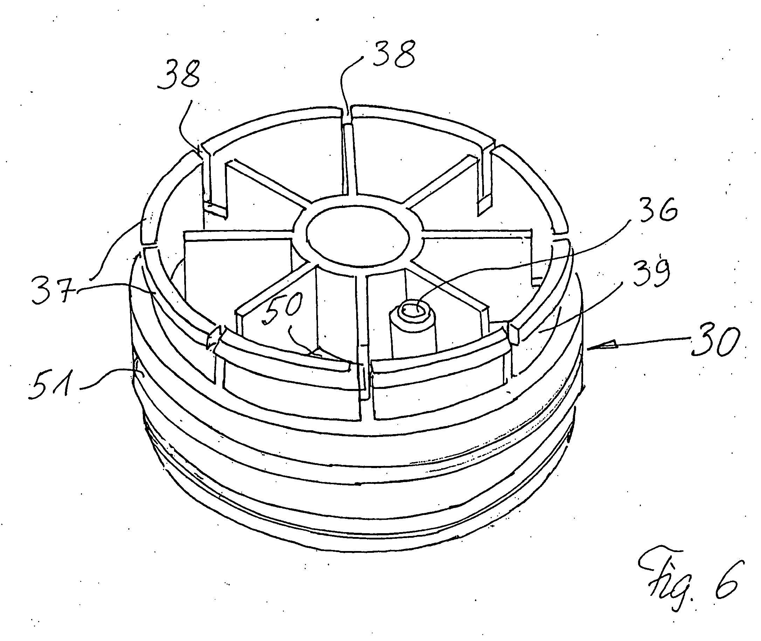

Der Kolben 3 umfasst einen Kolbenkörper 30, wie am besten aus Fig. 6 ersichtlich, ferner (siehe Fig. 1 und 4) einen großen O-Ring 31 zur Abdichtung am Hohlraum 10, einen kleinen O-Ring 32 zur Abdichtung an der Betätigungsstange 21, eine Filterscheibe 33, einen kleinen Klippring 34 und einen großen Klippring 35. Nach Einlegen der O-Ringe 31, 32 und der Filterscheibe 33 in entsprechende Aufnahmenuten am inneren Enden des Kolbenkörpers 30 (in Fig. 6 unten) werden die Klippringe 34, 35 auf den Kolbenkörper 30 gesteckt, um die O-Ringe und die Filterscheibe am Kolben festzulegen und zu befestigen. Am äußeren Ende (in Fig. 6 oben) weist der Kolben noch einen Sauganschlussstutzen 36 auf, der zu einem Raum innerhalb des Kolbens führt, an welchem die Filterscheibe 33 angelegt ist. Der Sauganschluss dient dazu, störende Luft in dem Mischraum 10 durch die Filterscheibe 33 hindurch abzusaugen, während das Mischgut im Mischguthohlraum durch die Filterscheibe 33 zurückgehalten wird.

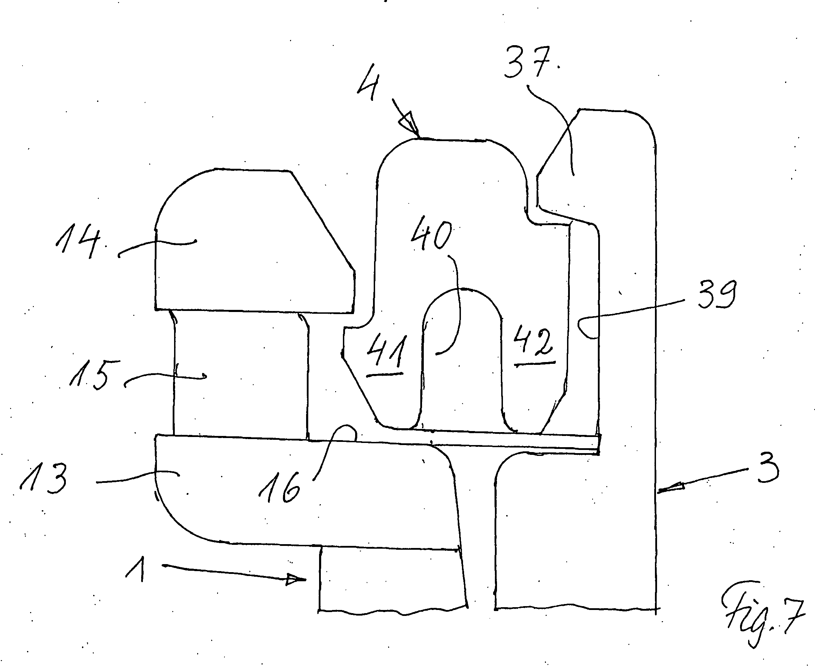

Der Rastring 4 dient zum zeitweiligen Befestigen des Kolbens 3 am Befüllende 11 des Kartuschenzylinders 1. Zu diesem Zweck weist der Rastring 4 radial außenseitige, ringförmige Rastmittel 41 und radial innenseitige, ringförmige Rastmittel 42 auf, die im Querschnitt des Rastringes als Hakenausbildung 41 bzw. als zylinderischer Ringabschnitt 42 mit Einführungsschräge erscheinen (siehe Fig. 3 und 7). Die Rastmittel 41, 42 sind durch eine Nut 40 voneinander getrennt und entwickeln dadurch eine federnde Nachgiebigkeit, die bei der Montage des Rastringes nützlich ist.

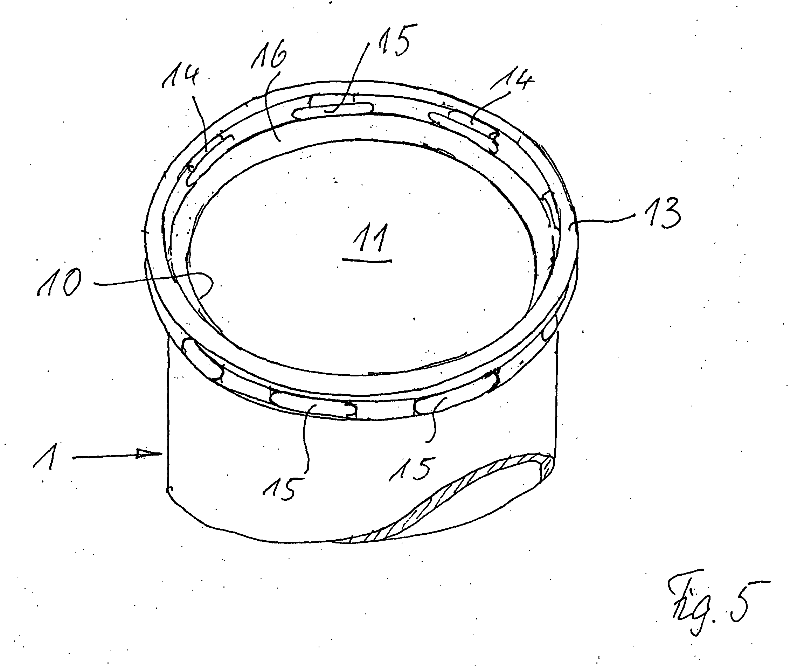

Fig. 5 zeigt einen Blick auf das Befüllende 11 des Kartuschenzylinders 1. Dieser weist einen Randflansch 13 auf, an dessen Innenseite sich ein Kranz von Gegenhaken 14 als Gegenrastmitteln zu den Hakenausbildungen 41 des Rastrings 4 befinden. Unterhalb der jeweiligen Gegenhaken 14 befinden sich schlitzförmige Schieberöffnungen 15, die zur Bildung der Unterseite der Gegenhaken 14 benötigt werden. Diese Schlitze 15 münden auf einem Aufsetzrand 16, der einen Anschlag für den Rastring 4 bildet.

Fig. 6 gewährt einen Blick auf das äußere Ende des Kolbenkörpers 30. Die in Fig. 6 oberen Wandteile des Kolbenkörpers sind als federnde, mit Einführungsschrägen versehene Rasthaken 37 ausgebildet, welche die Gegenrastmittel zu den innenseitigen Rastmitteln 42 des Rastrings 4 bilden. Die federnde Nachgiebigkeit der Rasthaken 37 wird durch die Anzahl und Tiefe von axialen Schlitzen 38 bestimmt, welche die in Fig. 6 obere Kolbenwand unterteilen. Unterhalb der Rasthaken 37 befindet sich eine umlaufende Nut 39 zur Aufnahme der Rastmittel 42 des Rastringes 4, wenn dieser an den Kolben 3 angekoppelt wird. Der Kolbenkörper 30 weist noch eine radiale Trennwand 50 auf, durch die der Sauganschlussstutzen 36 hindurch reicht und die auf die äußere Ringwand des Kolbens im Bereich einer Umfangsnut 51 stößt, die zur Vermeidung von Materialansammlung gebildet ist. Die Trennwand liegt außerhalb der Reichweite der Schlitze 38.

Fig. 7 zeigt das Zusammenwirken des Rastrings 4 mit dem Befüllende 11 des Kartuschenzylinders 1 und mit den Rasthaken 37 des Kolbens 3. Zur Montage wird der Rastring 4 auf die Rasthaken 37 des Kolbens 3 aufgesetzt und durch eine axial wirkende Kraft auf den Rastring zum Einschnappen in die Nut 39 hinter den Rasthaken 37 gebracht. Die radiale Ausdehnung des zylindrischen Ringabschnitts 42 mit konstruktiv zu wählender, größerer oder kleinerer. Überlappung mit dem Rasthaken 37 bestimmt die Haltekraft, mit der der Rastring 4 am Kolben 3 gehalten wird. Durch Aufsetzen der Hakenausbildungen 41 des Rastrings 4 auf der Einführungsschräge der federnden Gegenhaken 14 und axiales Zusammendrücken schnappen die Hakenausbildungen 41 hinter den Gegenhaken 14 ein und halten den Rastring 4 samt daran montiertem Kolben 3 am Befüllende 11 des Kartuschenzylinders 1. Auch hier sind die Montagekräfte dadurch abstimmbar, dass die radiale Abmessung der Hakenausbildungen 41 größer oder kleiner gewählt wird.

Für medizinische Anwendungen wird die Misch- und Appliziervorrichtung in sterilisierten, vormontierten Baueinheiten geliefert, nämlich der Kartuschenzylinder 1 mit angebrachtem Boden als Verschluss am Austrittsende 12, der Mischer 2 mit gefangen montiertem Kolben 3 samt aufgestecktem Rastring 4, die Komponenten des medizinischen Zements (Knochenzement) in entsprechenden Behältern, ein Einfülltrichter und ein Applizierrohr (Schnorchel), wobei diese Bauteile in einer Schale oder einem Beutel steril verpackt sind. Zu der Ausrüstung gehören noch eine Saugpumpe und eine Applikatorpistole zum Vorschieben des Kolbens und Herauspressen des Mischgutes aus dem Kartuschenzylinder.

Die Handhabung der Misch- und Appliziervorrichtung ist wie folgt:
Der Kartuschenzylinder wird mit dem zu mischenden Gut gefüllt. Die Saugpumpe wird am Saugstutzen 36 angeschlossen und der Mischer 2 samt Kolben 3 in das Befüllende des Kartuschenzylinders eingesteckt. Der angelegte Unterdruck ist bei der Montage des Kolbens 3 als Verschluss des Befüllendes behilflich, d. h. man braucht nicht viel Kraft auszuüben, um den Rastring mit seiner Hakenausbildung 41 hinter die federnden Gegenhaken 14 einschnappen zu lassen. Durch Evakuieren überschüssiger Luft und etwaiger Dämpfe aus dem Mischguthohlraum 10 wird das Mischgut zur Vermischung vorbereitet. Die Vermischung erfolgt durch Drehen und Hin- und Herschieben des Mischpaddels 22 innerhalb des Mischguthohlraums. Nach Durchmischen wird der Mischpaddel in Anlage an die Innenseite des Kolbens gebracht und die Betätigungsstange 21 so abgebrochen, dass der Mischguthohlraum verschlossen bleibt. Nunmehr kann der Kartuschenzylinder auf die Applikatorpistole aufgesetzt werden, der Verschlussboden wird entfernt und statt dessen das Applizierrohr am Austrittsende aufgeschraubt. Gegebenenfalls wird das Applizierrohr gekürzt. Nunmehr kann die Applikatorpistole betätigt werden, wobei Druck auf den Kolben 3 und Zug auf den Flansch 13 des Kartuschenzylinders ausgeübt wird. Dadurch wird die Haltekraft des Rastrings 4 überwunden, mit der der Kolben 3 am Befüllende 11 des Kartuschenzylinders gehalten wird. Der Kolben 3 zusammen mit dem in ihm steckenden, abgebrochenen Ende der Betätigungsstange 21 kann nunmehr durch die Applikatorpistole in Richtung auf das Austrittsende 12 des Kartuschenzylinders verschoben werden, um das Mischgut durch das Applizierrohr hindurch an geeigneter Stelle zu verbringen.

Es ist somit ersichtlich, dass die neue Misch- und Appliziervorrichtung mit dem Rastring 4 ein Halteelement zum zeitweiligen Befestigen des Kolbens am Befüllende aufweist, das vor Ansetzen der Applikationspistole nicht entriegelt werden braucht. Damit entfällt ein sonst beim Stand der Technik (EP 1 278 488 B1) benötigter Riegel sowie der Arbeitsgang der Betätigung und Entfernung des Riegels.

## Patentansprüche

1. Vorrichtung zum Mischen und Applizieren von Mischgut, insbesondere medizinischem Zement, umfassend:
einen Kartuschenzylinder (1) mit einem Mischguthohlraum (10), einem Befüllende (11) und einem Austrittsende (12), wobei das Befüllende (11) und das Austrittsende (12) sich an unterschiedlichen Enden des Kartuschenzylinders (1) befinden;
einen Mischer (2) mit einer Betätigungsstange (21), einem Mischpaddel (22) und einem Griff (23);
einen Kolben (3) zum Verschließen des Mischguthohlraums (10) am Befüllende (11) während des Mischens und zum Herauspressen des Mischgutes durch das Austrittsende (12) während des Applizierens; und
ein Halteelement zum zeitweiligen Befestigen des Kolbens (3) am Befüllende (11);
**gekennzeichnet durch** folgende Merkmale:
das Halteelement ist als Rastring (4) mit radial außenseitigen und innenseitigen Rastmitteln (41, 42) ausgebildet;
das Befüllende (11) des Kartuschenzylinders (1) weist erste Gegenrastmittel (14) auf, die mit den außenseitigen Rastmitteln (41) des Rastringes (4) zusammenwirken, wenn der Kolben (3) den Mischguthohlraum (10) verschließt;
der Kolben (3) weist zweite Gegenrastmittel (37) auf, die mit den innenseitigen Rastmitteln (42) des Rastringes (4) zusammenwirken, um den Kolben (3) während des Mischens zu halten und den Kolben (3) bei Beginn des Applikationsvorganges zur Verschiebung freizugeben.

2. Vorrichtung nach.Anspruch 1,
**dadurch gekennzeichnet, dass** die außenseitigen Rastmittel (41) des Rastringes (4) einen Ringabschnitt mit einer Hakenausbildung im Querschnitt aufweist, und dass die ersten Gegenrastmittel (14) des Kartuschenzylinders (1) einen Kranz von Gegenhaken bilden, die mit Einführungsschrägen zur erleichterten Montage des Rastringes (4) ausgebildet sind.

3. Vorrichtung nach Anspruch 2,
**dadurch gekennzeichnet, dass** der Kranz der Gegenhaken (14) auf der Innenseite eines Flanschrings (13) angebracht ist, der am Befüllende (11) des Kartuschenzylinders (1) angeordnet ist und sich mit einem Aufsetzrand (16) an den Mischguthohlraum (10) anschließt.

4. Vorrichtung nach Anspruch 3,
**dadurch gekennzeichnet, dass** jedem Gegenhaken (14) eine sich radial erstreckende Schlitzöffnung (15) in dem Flanschring (13) zugeordnet ist.

5. Vorrichtung nach einem der Ansprüche 1-4,
**dadurch gekennzeichnet, dass** die innenseitigen Rastmittel (42) des Rastrings (4) einen zylindrischen Ringabschnitt mit Einführungsschräge bilden, der von den außerseitlichen Rastmitteln (41) des Rastrings (4) durch eine Nut (40) getrennt ist, und
dass die zweiten Gegenrastmittel (37) des Kolbens (3) federnde, mit Einführungsschrägen versehene Rasthaken bilden, die aus Wandteilen des Kolbens (3) gebildet sind und eine Aufnahmenut (39) für den zylindrischen Ringabschnitt (42) des Rastrings (4) bestimmen.

6. Vorrichtung nach Anspruch 5,
**dadurch gekennzeichnet, dass** die federnden Rasthaken (37) voneinander durch axiale Schlitze (38) der äußeren Wand des Kolbens (3) getrennt sind, wobei die Anzahl und Länge der Schlitze (38) im Hinblick auf die gewünschte Federeigenschaft der Rasthaken (37) gewählt sind.

7. Vorrichtung nach Anspruch 6,
**dadurch gekennzeichnet, dass** der Kolben (3) eine radiale Trennwand (50) aufweist, die sich in einer größeren Entfernung von den Rasthaken (37) befindet, als er der Länge der Schlitze (38) entspricht, und dass der Kolben (3) in der Ebene der Trennwand (50) an seinem Außenumfang eine Nut (51) zur Vermeidung von Materialanhäufung aufweist.

## Claims

1. Device for mixing and applying mixed material, in particular medical cement, comprising:
a cartridge cylinder (1) having a mixed material cavity (10), a filling end (11) and an outlet end (12), wherein the filling end (11) and the outlet end (12) are located at different ends of the cartridge cylinder (1);
a mixer (2) having an actuating rod (21), a mixing paddle (22) and a grip (23);
a plunger (3) for closing the mixed material cavity (10) at the filling end (11) during mixing and for urging the mixed material out through the outlet end (12) during application; and
a holding element for temporarily securing the plunger (3) to the filling end (11);
**characterised by** the following features:
the holding element is formed as a latching ring (4) having radially outer and inner latching means (41, 42);
the filling end (11) of the cartridge cylinder (1) comprises first counter-latching means (14) which cooperate with the outer latching means (41) of the latching ring (4) when the plunger (3) closes the mixed material cavity (10); and
the plunger (3) comprises second counter-latching means (37) which cooperate with the inner latching means (42) of the latching ring (4), in order to hold the plunger (3) during mixing and to release the plunger (3) for displacement at the beginning of the application procedure.

2. Device as claimed in claim 1, **characterised in that** the outer latching means (41) of the latching ring (4) comprise a ring portion having a hook formation in cross-section, and that the first counter-latching means (14) of the cartridge cylinder (1) form an annulus of counter hooks which are formed with insertion bevels to facilitate mounting of the latching ring (4).

3. Device as claimed in claim 2, **characterised in that** the annulus of counter hooks (14) is provided on the inner side of a flange ring (13) which is disposed at the filling end (11) of the cartridge cylinder (1) and adjoins the mixed material cavity (10) by means of a positioning edge (16).

4. Device as claimed in claim 3, **characterised in that** each counter-hook (14) is allocated a radially extending slit opening (15) in the flange ring (13).

5. Device as claimed in any one of claims 1-4, **characterised in that** the inner latching means (42) of the latching ring (4) form a cylindrical ring portion which has an insertion bevel and is separated from the outer latching means (41) of the latching ring (4) by means of a groove (40), and that the second counter-latching means (37) of the plunger (3) form resilient latching hooks which are provided with insertion bevels, are formed from wall parts of the plunger (3) and define a receiving groove (39) for the cylindrical ring portion (42) of the latching ring (4).

6. Device as claimed in claim 5, **characterised in that** the resilient latching hooks (37) are separated from one another by means of axial slits (38) in the outer wall of the plunger (3), wherein the number and length of the slits (38) are selected with regard to the desired resilient characteristic of the latching hooks (37).

7. Device as claimed in claim 6, **characterised in that** the plunger (3) comprises a radial separating wall (50) which is located at a greater distance from the latching hooks (37) than it corresponds to the length of the slits (38), and that in the plane of the separating wall (50) the plunger (3) comprises on its outer circumference a groove (51) for obviating material accumulation.

## Revendications

1. Dispositif pour mélanger et appliquer un produit mélangé, en particulier du ciment médical, comprenant :
un cylindre à cartouche (1) doté d'une cavité pour le produit mélangé (10), d'une extrémité de remplissage (11) et d'une extrémité de sortie (12), l'extrémité de remplissage (11) et l'extrémité de sortie (12) se trouvant sur différentes extrémités du cylindre à cartouche (1) ;
un mélangeur (2) doté d'une barre d'actionnement (21), d'une ailette de mélange (227) et d'une poignée (23) ;
un piston (3) pour la fermeture de la cavité de produit mélangé (10) sur l'extrémité de remplissage (11) pendant le mélange et pour la sortie par compression du produit mélangé par l'extrémité de sortie (12) pendant l'application ; et
un élément de retenue pour la fixation temporaire du piston (3) sur l'extrémité de remplissage (11) ;
**caractérisé par** les caractéristiques suivantes :
l'élément de retenue est conçu sous forme de bague d'arrêt (4) avec des moyens d'arrêt (41, 42) côté extérieur et côté intérieur sur un plan radial ;
l'extrémité de remplissage (11) du cylindre à cartouche (1) présentant des contre-moyens d'arrêt (14), qui coopèrent avec les moyens d'arrêt (41) côté extérieur de la bague d'arrêt (4) lorsque le piston (3) ferme la cavité du produit mélangé (10) ;
le piston (3) présente des seconds contre-moyens d'arrêt (37), qui coopèrent avec les moyens d'arrêt (42) côté intérieur de la bague d'arrêt (4), afin de maintenir le piston (3) pendant le mélange et de libérer le piston (3) au début de l'opération d'application pour le déplacement.

2. Dispositif selon la revendication 1,
**caractérisé en ce que** les moyens d'arrêt (41) côté extérieur de la bague d'arrêt (4) présentent un tronçon de bague avec une conception en crochet en section transversale, et **en ce que** les premiers contre-moyens d'arrêt (14) du cylindre à cartouche (1) forment une couronne de contre-crochets, qui sont conçus avec des chanfreins d'introduction pour faciliter le montage de la bague d'arrêt (4).

3. Dispositif selon la revendication 2,
**caractérisé en ce que** la couronne des contre-crochets (14) est placée sur le côté intérieur d'une bague à bride (13), qui est disposée sur l'extrémité de remplissage (11) du cylindre à cartouche (1) et se raccorde avec un bord rapporté (16) à la cavité de produit mélangé (10).

4. Dispositif selon la revendication 3,
**caractérisé en ce qu'**une ouverture de fente (15) s'étendant radialement dans la bague à bride (13) est attribuée à chaque contre-crochet (14).

5. Dispositif selon l'une quelconque des revendications 1 à 4,
**caractérisé en ce que** les moyens d'arrêt (42) côté intérieur de la bague d'arrêt (4) forment un tronçon de bague cylindrique avec chanfrein d'introduction, qui est séparé des moyens d'arrêt (41) côté extérieur de la bague d'arrêt (4) par une rainure (40), et **en ce que** les seconds contre-moyens d'arrêt (37) du piston (3) forment des crochets d'arrêt montés sur ressort et dotés de chanfreins d'introduction, qui sont formés de parties de paroi du piston (3) et déterminent une rainure de logement (39) pour le tronçon de bague (42) cylindrique de la bague d'arrêt (4).

6. Dispositif selon la revendication 5,
**caractérisé en ce que** les crochets d'arrêt (37) montés sur ressort sont séparés les uns des autres par des fentes (38) axiales de la paroi extérieure du piston (3), le nombre et la longueur des fentes (38) étant choisis par rapport à la propriété de ressort choisie des crochets d'arrêt (37).

7. Dispositif selon la revendication 6,
**caractérisé en ce que** le piston (3) présente une cloison de séparation (50) radiale, qui se trouve à une distance des crochets d'arrêt (37) plus grande que la longueur des fentes (38), et **en ce que** le piston (3) présente dans le plan de la paroi de séparation (50) sur son pourtour extérieur une rainure (51) destinée à éviter l'accumulation de matériau.
